# EUROPEAN PATENT SPECIFICATION

(11) **EP 2 552 937 B1**
(45) Date of publication and mention of the grant of the patent: **17.01.2018**
(21) Application number: 10848810.7
(22) Date of filing: 17.05.2010
(51) Int. Cl.: C07K 1/36, C07K 7/56

(54) **A PROCESS FOR PURIFICATION OF PNEUMOCANDIN**
VERFAHREN ZUR AUFREINIGUNG VON PNEUMOCANDIN
PROCÉDÉ DE PURIFICATION DE LA PNEUMOCANDINE

(30) Priority: 29.03.2010 IN 861CH2010
(43) Date of publication of application: 06.02.2013
(73) Proprietor: Biocon Limited, Bangalore 560 100 Karnataka (IN)
(72) Inventor: RASTOGI, Kushal, Uttar Pradesh (IN); SANTAN, Onkar, Prakash, Nashik 422 009 Maharashtra (IN); PATIL, Nitin, Sopanrao, Karnataka (IN); MENDHE, Rakesh, Bhaiyyaram, Maharashtra (IN)
(74) Representative: Michalski Hüttermann & Partner Patentanwälte mbB
(86) International application number: PCT/IN2010/000313
(87) International publication number: WO 2011/121599

(56) References cited:
- WO-A1-2008/048627
- WO-A1-2009/142761
- WO-A2-2005/026323
- US-A1- 2002 028 916
- OSAWA A E ET AL: "Purification of pneumocandins by preparative silica-gel high-performance liquid chromatography", JOURNAL OF CHROMATOGRAPHY, ELSEVIER SCIENCE PUBLISHERS B.V, NL, vol. 831, no. 2, 29 January 1999 (1999-01-29), pages 217-225, XP004156148, ISSN: 0021-9673, DOI: 10.1016/S0021-9673(98)00936-4
- ROUSH D J ET AL: "Preparative high-performance liquid chromatography of echinocandins", JOURNAL OF CHROMATOGRAPHY, ELSEVIER SCIENCE PUBLISHERS B.V, NL, vol. 827, no. 2, 11 December 1998 (1998-12-11), pages 373-389, XP004153871, ISSN: 0021-9673, DOI: 10.1016/S0021-9673(98)00804-8
- NTI-GYABAAH J ET AL: "Normal phase high-performance liquid chromatography of pneumocandins: in situ modification of silica with L-proline to separate structural analogues", BIOTECHNOLOGY PROGRESS, AMERICAN INSTITUTE OF CHEMICAL ENGINEERS, US, vol. 22, no. 2, 1 March 2006 (2006-03-01), pages 538-546, XP002479955, ISSN: 8756-7938, DOI: 10.1021/BP050319T [retrieved on 2006-02-11]

## Description

### FIELD OF THE INVENTION

The present invention is in relation to a purification process of a secondary metabolite produced by fermentation route. The process involves the selective removal of impurities at various stages of washings, charcoalization followed by crystallization. The product is closely related to the class of echinocandins and is found to be a potent **antifungal** compound and a key ingredient in the synthesis of antifungal drugs.

### BACKGROUND OF THE INVENTION

The instant invention describes a novel process for the purification of a naturally occurring secondary metabolite obtained from fermentation route. The product is closely related to echinocandins and is known to be a key intermediate to make antifungal agents. In particular, Pneumocandins are discussed in detail here. It is a cyclic hexapeptide with multiple hydroxyl groups and a hydrophobic dimethylmyristate tail connected via an amide bond to the alpha amino group of the hydroxylated ornithine residue. According to Schwartz et al. (R.E. Schwartz, D.F. Sesin, H. Joshua, K.E. Wilson, A.J. Kempf, K.E. Golden, D. Kuehner, P. Gailliot, C. Gleason, R. White, E. Inamine, G. Bills, P. Salmon, L. Zitano, Pneumo candins from Zalerion arboricola. I. Discovery and isolation, J. Antibiotics 45 (1992) 1853) and Bills et al. (G.F. Bills, G. Platas, F. Pelaez, P. Masurekar, Reclassification of a pneumocandin-producing anamorph, Glarea lozoyensis gen. et sp. nov., previously identified as Zalerion narboricola, Mycological Research 102 (1998)), Pneumocandin B₀ can be produced by fermentation of *Glarea lozoyensis (Zalerion arboricola).* According to Hensens et al. (O.D. Hensens, J.M. Liesch, D.L. Zink, J.L. Smith, C.F. Wichman, R.E. Schwartz, J. Antibiotics 45 (1992) 1875 and A. Adeferati, O. Hensens, E.T.T. Jones, J. Tkacz, J. Antibiotics 45 (1992) 1953), the organism can produce other echinocandins in addition to the desired product Pneumocandin B₀ including its isomers Pneumocandin A₀ and C₀.

International patent application WO 2008/048627 discloses a method of preparing and purifying echinocandin-type compounds, such as pneumocandin B₀. Osawa et al. (Journal of Chromatography A (1999) 831, 217-225), Roush et al. (Journal of Chromatography A (1998) 827, 373-389) and Nti-Gyabaah et al. (Biotechnology Progress (2002) 22, 2, 538-546) relate to the purification of pneumocandin B₀ using HPLC. International patent application WO 2005/026323 relates to a novel stationary phase and its use for purifying a peptide or lipopeptide in liquid chromatography. Structures of Pneumocandins are shown below.

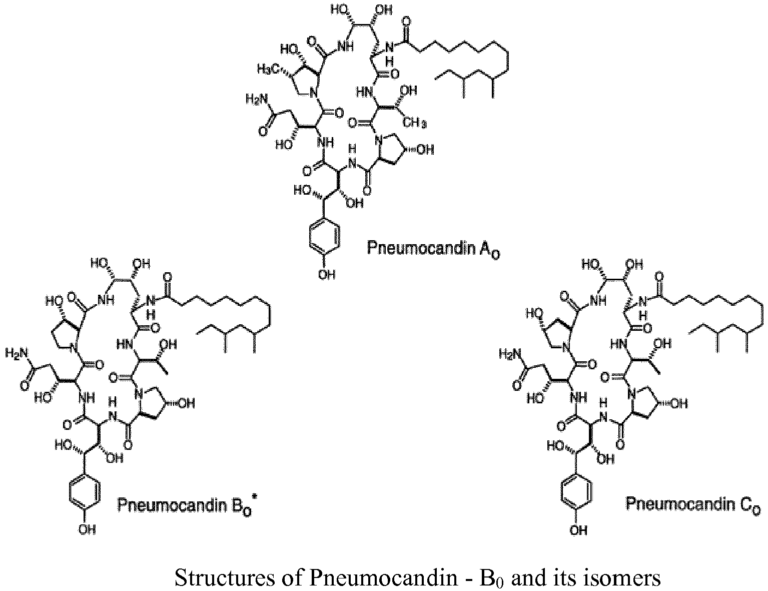

The process followed for the purification of crude Pneumocandin-B₀ and related compounds involves solvent-solvent extractions, repeated column purifications and crystallizations which are tedious and need to look into simple and robust process. The instant process is simple and ease to operate. The claimed process involves a novel purification route involving washings with immiscible solvents or water, removal of UV inactive colored impurities and product selective crystallization. Process - 1 has been found to have higher yields, better purity and lower raw material costs. The instant process in turn results in a purity of more than 90%.

### STATEMENT OF THE INVENTION

The present invention provides a process for purification of pneumocandin B₀, having one or more polar impurities and one or more non-polar impurities. The process comprises extraction of product from the fermentation broth using a suitable solvent and partially concentrating, washing with immiscible solvent, charcoalization, concentrating and filtering, crystallizing the filtrate to obtain a solid, loading the solids into a column with an adsorbent, eluting impurities by passing one column volume of water through the column, eluting the product from the column with one or more suitable solvents to obtain eluted pneumocandin B₀, concentration of product rich fractions and crystallization of the eluted pneumocandin B₀ to obtain the pneumocandin B₀. The suitable solvent used in extraction of product from the fermentation broth is selected from n-butanol, sec-butanol, tertiary-butanol and n-propanol. The adsorbent of the column is alumina or silica gel.

### BRIEF DESCRIPTION OF THE ACCOMPANYING DRAWINGS:

**FIGURE 1:** XRD of Pneumocandin - B₀.

### DETAILED DESCRIPTION OF THE INVENTION

A process for the purification of pneumocandin having one or more polar impurities and one or more non-polar impurities disclosed herein comprises;
a) extraction of product from fermentation broth using suitable solvent and partially concentrated,
b) washing with immiscible solvent,
c) charcoalization,
d) concentration and filtration,
e) loading the solids obtained from step (d) in a column with an adsorbent,
f) eluting with suitable solvents,
g) concentration of product rich fractions and
h) crystallization.

Pneumocandin is Pneumocandin-B₀.

A suitable solvent for extraction of product from the fermentation broth, used in the present invention, is selected from a group comprising n-butanol, sec-butanol, tertiary-butanol and n-propanol.

In an embodiment according to the present invention, the solvent is n-butanol.

In still another embodiment of the present invention, the immiscible solvent is selected from a group comprising water, pet-ether and cyclohexane.

In still another embodiment of the present invention, the solvent is water.

In still another embodiment of the present invention, crystallization is carried out by the solvent-antisolvent method.

In still another embodiment of the present invention, crystallization is carried out by the solvent-antisolvent method.

According to the present invention, the solvent is selected from a group comprising methanol, n-propanol, iso-propanol, n-butanol, sec-butanol, tertiary-butanol and mixture thereof.

In still another embodiment of the present invention, the anti solvent is selected from a group comprising acetone, acetonitrile, pet-ether, cyclohexane, ethyl acetate, water and heptane.

In still another embodiment of the present invention, the solvent is n-butanol.

In still another embodiment of the present invention, the anti solvent is acetone.

According to the present invention, the adsorbent is selected from a group comprising alumina and silica gel.

In another embodiment of the present invention, the adsorbent is alumina.

The solvent for elution can be selected from a group comprising methanol, n-propanol, iso-propanol, n-butanol, sec-butanol, tertiary-butanol, pentane, hexane, heptane, octane, ethyl acetate water and mixture thereof.

According to the present invention, polymorph obtained is amorphous as shown in figure-1.

In still another embodiment of the present invention, wherein purity is more than 90%.

The process described herein relates to a purification process of the cyclic lipopeptide type molecules for example echinocandin family having one or more polar impurities and one or more non-polar impurities, comprising following steps:
a) extraction of product from fermentation broth using suitable solvent and partially concentrated,
b) washing with immiscible solvent,
c) charcoalization,
d) concentration and filtration,
e) loading the solids obtained from step (d) in to a column preloaded with an adsorbent,
f) eluting with suitable solvents,
g) concentration of product rich fractions and
h) crystallization.

The process described herein is aimed at purification of naturally occurring secondary metabolite obtained from fermentation route. The process can be used in general for cyclo - peptide type molecules for example echinocandins. In particular the process aims at purification of Pneumocandin - B₀ which is a key product to obtain caspofungin di-acetate, a known antifungal agent, through synthetic route. The instant process involves selective removal of impurities, where the product remains in one solvent and is treated with immiscible solvents for back wash; further it is treated with activated charcoal to remove significant amount of UV inactive colored impurities present from the start. This is followed by selective crystallization of the product, while impurities largely remain in the mother liquor, and the product precipitates. The first crystallization of the process results in amorphous form of Pneumocandin-B₀ with moderate purity (75-85%).

Further these crystals involves binding of product along with impurities onto a adsorbent. Typically N-alumina is used, but other variants of alumina or silica can also be used. After this, elution is carried out with a solvent composition which is more selective towards impurities. This purification results in the removal of other impurities and particularly A₀ in this step. This is followed by a solvent composition which elutes moderate purity product, where some of other remaining impurities are removed. Further, elution is carried out with a product selective solvent which elutes high purity product. These high purity fractions are pooled and concentrated and crystallized to get high purity Pneumocandin-B₀ in solid form (>90%).

In particular, purification of Pneumocandin - B₀ of the structure shown above is discussed.

The purification process involves extraction of the product from fermentation broth using suitable solvent, mostly alcohols viz. propanol, iso butanol, t-butanol, n-butanol. The Pooled extract layer is concentrated to about 30-50 g/kg stage under vacuum at 45-50 °C. The next step is to remove polar and various other impurities present in the concentrated extract. This is accomplished using a solvent or solvent composition which is immiscible. As n-butanol is present in partially concentrated extracts, water can be taken for back wash. Typically -2-3 volumes of water w.r.t. concentrated pooled extract is added and is mixed well and allowed for settling. The n-butanol layer separates from the aqueous layer along with product, while large quantities of impurities are retained in the aqueous layer. ∼10 - 35% of purity increase can be obtained depending upon impurities in the fermentation broth. Water wash can be repeated to achieve better possible purity. This n-butanol layer is diluted by adding an additional quantity of n-butanol to make the product concentration -5-10 g/kg and treated with activated charcoal. Typically 0.5:1 - 5:1 (w/w) charcoal is used. The suspension is stirred well and filtered through celite bed followed by bed wash. UV inactive colored impurities are adsorbed in activated charcoal, which further gets adsorbed on celite bed providing removal of non UV impurities. Also some UV active impurities get adsorbed by celite, giving a chromatographic purity increase of about 10%. The n-butanol product layer is concentrated to about 30 g/kg stage.

The next step of purification involves crystallization. The crystallization described herein utilizes controlled addition of anti-solvent, and cooling both. The solvent can be any of among methanol, propanol, iso-propanol, n-butanol, t-butanol, iso-butanol etc., and the antisolvent can be any of among acetone, acetonitrile, ethyl acetate, water etc., whereas preferably acetone is used.

Concentrated pooled product layer after purification steps as discussed above, is taken in a jacketed vessel for crystallization. Slow addition of antisolvent is initiated at room temperature. The flow rate is adjusted using a pump in such a way that 5 volumes of antisolvent is added over 4 -6 hours. After 2-3 volumes addition of antisolvent, solution reaches close to saturation point at RT room temperature. At this moment, cooling is started and the temperature is brought down to 0-10 °C while continuing addition of antisolvent. The crystallization step is sensitive towards initial product concentration, addition rate of antisolvent and cooling. Fast addition may yield to precipitate the impurities too, while slow addition may yield to wet paste instead of solid powder. Vacuum filtration is used to separate the solids from the mother liquor. The solid product is dried under vacuum at 40 °C for 24 hours. Depending upon the input purity of material, successive crystallization can be done to achieve further purification, but it is observed that purification beyond 75-85% becomes extremely difficult.

Solids obtained from the above step with purity 75-85% of Pneumocandin - B₀ co-exists with its isomers A₀ and C₀ and other closely related impurities. Separation of these impurities is difficult using simple unit operations like crystallization. Repetitive crystallization offers a very slight increase in purity at a significant loss of product. Conventionally it is required to have chromatographic preparative runs to isolate the B₀. Also, it is known that only normal phase chromatography with very specific mobile phase gives resolution between A₀, B₀, C₀ and other related impurities. The instant process further involves a novel method to further purification to obtain high purity (greater than 90%) of Pneumocandin B₀. The process involves loading of moderate purity (∼75-80%) Pneumocandin - B₀ onto an adsorbent. The adsorbent can be chosen among variants of Alumina or silica gel. Neutral alumina is particularly discussed in detail here. At this step, product along with impurities binds to the adsorbent bed. After this, selective removal of impurities is carried out by varying composition of the solvents. First a solvent composition selective towards impurities is chosen to selectively elute impurities rich fractions, and then a composition selective towards product is chosen to selectively elute high purity product fractions.

Solvents for the elution were chosen among methanol, n-propanol, iso-propanol, n- butanol, t-butanol, sec-butanol, ethyl acetate, hexane, heptane and water etc. The study showed that water-rich eluent is highly selective for related impurities. It was observed that water rich eluent with small quantities of methanol is more selective towards A₀ isomer and iso-propanol rich solvent with hexane, (typically 80/20 v/v); is more selective towards C₀. Though it was found that certain composition of ethyl acetate, methanol water was more selective towards related impurities.

Different ratios of N-alumina with respect to product were tried and 30:1 (w _{Alumina}/w _{product}) ratio was found to be optimum for better yields and purity. Higher ratio of alumina requires more quantity of solvents to elute. The same percentage of product as in case of 30:1. Lower ratios of N-alumina result into loss of product.

To start with, N-alumina (30:1 w/w) is packed in a glass column to make a uniform bed. At production scale, a nutsche filter can be used for bed packing. Crystals obtained from the previous step are dissolved in methanol to make a product concentration -15-40 g/L. This is loaded onto the N-alumina bed. A small amount of flow through is obtained, which shows only 1-2% of product loaded.

After this, selective elution of impurities is initiated. Typically one column volume of water is passed through the bed, which takes out mainly related impurities and some A₀. This is followed by selective elution of other impurities. Typically a gradient mixture of methanol-water is used for 5-8 column-volumes, which takes almost all of related impurities and a greater extent of A₀. Finally product -B₀ with moderate purity (∼80-84%) elutes and is collected separately. After selective elution of these impurities, 100% methanol is used for elution. It is most selective towards product as almost all related impurities and a significant amount of A₀ is already eluted, the product starts eluting with very high purity. 10-15 such fractions are eluted and collected separately. The purity of initial fractions ranges between 88-90%, while later fractions purity varies between 90-95%. These high purity fractions are pooled and concentrated to about 60-100 g/kg stage. Further this concentrate is crystallized using slow addition of acetone as antisolvent at a temperature of 0-10 °C as discussed in earlier.

The details of the method are exemplified with the help of examples given below. However it should not be construed that the scope disclosure is limited to the examples.

### EXAMPLES :

20 kg of fermentation broth containing about 31 gm product - Pneumocandin B₀ was extracted using 8 Kg of n-butanol.

### Example-1

853 gm of n-butanol extract layer consisting of 4.3 gm of product at a purity of 22.8% was taken and concentrated to 30 g/kg stage. This concentrate was washed with water 1:1 (w/w) basis. The n-butanol layer obtained showed 4.1 gm of product with purity 44.9%. This n-butanol layer was pooled with another n-butanol layer (344 gm) with 4.93 gm of product at purity 26.7%. This pooled n-butanol layer was concentrated to -30 g/kg stage followed by second stage water wash at 3:1 (w/w) basis. The n-butanol layer separated post water wash weighed 252 gm with product 8.4 gm (92.8%) at purity 60%. Aqueous layer weighed 923 gm with 0.407 gm (4.5%) product only at purity of 2.7%.

35 gm n-butanol layer was taken out of 252 gm n-butanol obtained above. Analysis showed 1.15 gm of product at concentration of ∼33g/kg and purity ∼60%. 0.5 gm of activated charcoal was added to this and was stirred for 1 hour. Separately 15 gm of celite was taken and slurry was made using n-butanol. Bed of celite was packed on Buchner funnel and charcoal suspension was loaded on this celite bed so that charcoal along with UV-inactive impurities gets adsorbed on the celite surface. Filtrate (140 ml) consisted of 1.1 gm (95%) with purity 70.1%. Further bed was given wash with n-butanol (50ml) which showed 0.078 gm (∼5%) at purity 68.5%. These two were pooled (190ml) and used for crystallization.

Pooled filtrate and bed wash as obtained above (1.15 gm of product at purity ∼69%) was concentrated to -30 g/kg stage i.e. -40 ml. Acetone was added drop by drop to the n-butanol-product solution. Flow rate of acetone was kept at 0.66 ml/min. After 3 volumes of addition of acetone, cooling of reaction mass was started. Temperature was maintained between 8-10 °C. At this stage, solution became supersaturated and product started precipitating. Further addition of acetone was continued till 320 ml of acetone was added. Finally product was filtered out. The final product was brownish white in color. HPLC analysis showed **1.017 gm (88%)** of product with purity **78.6%.** XRD analysis showed it to be complete amorphous form.

### Example-2

To make the purification process robust, crystallization at lower purity of starting material was studied. Lower purity at crystallization stage may arise due to presence of greater percentage of impurities at the extract stage, or due to improper treatment at washing and/or other purification steps. It has been observed that in such a case additional crystallization may be required.

240 gm of n-butanol extract layer consisting of 4.97 gm of product at purity of 22.8% was taken. This was concentrated to product concentration of ∼30g/kg stage and was given a water wash with 1:1 (w/w) basis. The n-butanol layer post water wash showed 4.85 gm (97.6%) of product with a purity of 38%. This was diluted to 395 gm with n-butanol to make product concentration ∼10g/kg.

For charcoalization, 0.5:1 (w/w) of charcoal was taken and mixed with the product for ∼1hr. This was filtered on celite bed using 15:1 (w/w) of celite. 95% product was obtained in the filtrate and 5% in the bed-wash.

Filtrate and bed wash were pooled and crystallized similar to Example-1. Product (solid) obtained after the 1^{st} crystallization showed 3.56 gm of product with a purity of 58.5%. Post 2^{nd} crystallization, 2.84 gm product was obtained with 67 % purity. It required a 3^{rd} crystallization to achieve 81.23 % purity with final 1.98 gm product.

It has been observed that crystallization is one of the most critical step of purification **Process-1.** Antisolvent addition rate, quantity, introduction of cooling, initial load concentration and purity, nature of impurities all impact the performance and hence the results of crystallization. For example, at very high rate of antisolvent, product along with impurities precipitates, so purification does not happen that good. Further at fast antisolvent addition, more non UV-impurities precipitates, which may not be detected in HPLC purity but visibly looks very dark. Similarly, if cooling is started at very early stage when product is concentrated, precipitation starts very quickly resulting into similar phenomenon as discussed with faster antisolvent addition. Further, if addition rate is very slow, it does not crystallizes in a proper way and yields in a cake formation or fluffy mass on filtration instead of fine powder. It has also been studied that crystallization at 0-3 °C gives higher yields as compared to 8-10 °C due to further decreased solubility of product.

### EXAMPLE: PROCESS 2

The product Pneumocandin-B₀ obtained from process-1 was used for further purification to obtain high purity (>90%) of Pneumocandin - B₀. 1.5 gm of solid product was taken and was dissolved in methanol, such that the product concentration becomes ∼25g/L. The load was analyzed by HPLC and % area of product and impurities were as following: - Impurity T (RRT 0.33) : 0.68%, A₀ : 5.57%, B₀ : 77.99%, Related Impurity - R(RRT 1.05) : 4.42%, C₀ : 5.97% .

45 gm of N-alumina was taken and the bed was packed in a glass column. Bed dimensions were - diameter: 4 cm, height : 3.5 cm. The product was loaded onto this bed and the flow through was collected. The flow through showed only 1.3% of the product with a purity of 76.92%.

Now selective elution of impurities was carried out as shown in FIGURE-2. At first, 1 column volume elution with 100% water is carried out. This takes mainly impurity T (RRT 0.33) and impurity A₀, while little of product. HPLC analysis showed 5.57% of T, 26.23% of A₀ and 41.5% of B₀, while product loss was only 4.6%. %Area of R and C₀ was 1.2% & 2.5% respectively.

This was followed by elution with methanol/water 25/75% (v/v). A typical composition of a fraction was ∼8% T, ∼12% A₀, ∼60% B₀, ∼2% R and ∼4% C₀, while total product loss was only 1.5% in 6 column volumes.

Next, one column volume was eluted with methanol-water 50/50% (v/v). This favours the elution of other impurities along with the product, resulting in moderate purity of the product. Fraction analysis by HPLC showed ∼8% A₀, 83.88% B₀ and 3% R, while T and C₀ were almost absent. Product loss in this fraction was only 0.51%.

Next 15 column volumes were eluted with 100% methanol and were collected separately. First fraction had 85.08% purity and second fraction had 86.5% purity. Fractions 3-5 had purity of ∼88.3%, fractions 6-10 had purity of ∼90-91.8% while 11-14 had 92-93.7% purity. After this, decreasing trend of purity was observed. Fraction 15 showed 91.99% purity.

Pooled fractions 1-14 showed in total 54.6% of the product with purity 90.21%. These pooled fractions were concentrated to -25 ml and concentrate was transferred to a jacketed vessel. Acetone was used as antisolvent and crystallization was carried out as described earlier. Final product was filtered and dried under vacuum as described above. The final product was white in color and complete amorphous in form. HPLC analysis showed 91.37% purity of product - Pneumocandin B₀.

Although **Process**-**1** and **Process-2** are described for product Pneumocandin-B₀, it is understood that such techniques can be used for purification of other molecules obtained from fermentation, which are similar to lipo-peptides, particularly echinocandins family. The keys to the invention in Process-1 & Process-2 are the selective removal of impurities, while the product remains in one solvent from start to end. Process-1 has advantages over reported purification processes in terms of avoiding of repetitive back extraction and concentration steps with or without liquid-liquid extraction, which involves higher operating and raw materials cost along with significant losses of product. Process-1 discloses controlled crystallization of product, which selectively precipitates the product. Process-1 discloses a novel way of purification where product remains in only one solvent from start to end, which improves yield, eases recovery of solvents as well as reduces the raw material costs. It has been found that process-1 has an overall yield of 55-70% (with respect to broth) with final product purity 75-82%. Process-2 has an overall yield of 45-55% with final product purity 90-93%.

## Claims

1. A process for purifying pneumocandin B₀, having one or more polar impurities and one or more non-polar impurities, said process comprising the acts of:
a) extracting the product from the fermentation broth by extracting the same with a suitable solvent and partially concentrating, wherein the suitable solvent is an alcohol selected from n-butanol, sec-butanol, tertiary-butanol and n-propanol;
b) washing with immiscible solvent;
c) charcoalizing;
d) concentrating and filtering;
e) crystallizing the filtrate to obtain a solid;
f) loading the solid into a column with an adsorbent selected from alumina or silica gel;
g) eluting impurities by passing one column volume of water through the column;
h) eluting the product from the column with one or more solvent(s) to obtain eluted pneumocandin Bo; and
i) concentrating and crystallizing the eluted pneumocandin B₀ to obtain the pneumocandin B₀.

2. The process as claimed in claim 1, wherein the immiscible solvent is selected from a group comprising water, pet-ether and cyclohexane.

3. The process as claimed in claim 2, wherein the immiscible solvent is water.

4. The process of any of claims 1 - 3, wherein the crystallizing is carried out by the solvent/anti-solvent method.

5. The process as claimed in claim 4, wherein the solvent used in the solvent/anti-solvent method is selected from a group comprising methanol, n-propanol, iso-propanol, n-butanol, sec-butanol, tertiarybutanol and any combinations thereof.

6. The process as claimed in claim 4, wherein the antisolvent used in the solvent/anti-solvent method is selected from a group comprising acetone, acetonitrile, pet-ether, cyclohexane, ethyl acetate, water and heptane.

7. The process as claimed in claim 1, wherein the adsorbent is neutral alumina.

8. The process as claimed in claim 1, wherein the one or more solvents for eluting the product is/are selected from a group comprising methanol, n-propanol, iso-propanol, n-butanol, sec-butanol, tertiary-butanol, pentane, hexane, heptane, octane, ethyl acetate, and any combination of n-propanol, iso-propanol, n-butanol, sec-butanol, tertiary-butanol, pentane, hexane, heptane, octane, ethyl acetate, and water.

9. The process as claimed in claim 1, wherein the suitable solvent is n-butanol, wherein washing with immiscible solvent comprises adding n-butanol to obtain a product concentration of 5-10 g/kg, charcoalizing comprises stirring, and filtering is done through a celite bed.

10. The process of claim 1, comprising applying a gradient mixture of water/methanol for 5-8 column volumes after passing one column volume of water through the column.

11. The process of claim 1 or 10, comprising eluting pneumocandin B₀ from the column with methanol.

12. The process of claim 11, wherein the methanol is 100% methanol.

13. The process as claimed in claim 1, wherein the pneumocandin B₀ is amorphous pneumocandin B₀.

## Patentansprüche

1. Verfahren zur Reinigung von Pneumocandin B₀, das eine oder mehrere polare Verunreinigungen und eine oder mehrere unpolare Verunreinigungen hat, wobei das Verfahren die Schritte aufweist:
a) das Produkt aus dem Fermentationsmedium extrahieren, indem extrahieren desselben mit einem geeigneten Lösungsmittel und teilweise konzentrieren erfolgt, wobei das geeignete Lösungsmittel ein Alkohol ist, der aus n-Butanol, sec-Butanol, tert-Butanol und n-Propanol ausgewählt ist,
b) mit einem nicht mischbarem Lösungsmittel waschen,
c) mit Aktivkohle behandeln,
d) konzentrieren und filtrieren,
e) das Filtrat kristallisieren, so dass ein Feststoff erhalten wird,
f) den Feststoffe auf eine Säule mit einem Adsorptionsmittel laden, das aus Aluminiumoxid oder Silicagel ausgewählt ist,
g) Verunreinigungen eluieren, indem ein Säulenvolumen Wasser durch die Säule geleitet wird,
h) das Produkt von der Säule mit einem oder mehreren Lösungsmittel(n) eluieren, so dass eluiertes Pneumocandin B₀ erhalten wird, und
i) das eluierte Pneumocandin B₀ konzentrieren und kristallisieren, so dass das Pneumocandin B₀ erhalten wird.

2. Verfahren nach Anspruch 1, wobei das nicht mischbare Lösungsmittel aus einer Gruppe ausgewählt ist, die Wasser, Petrolether und Cyclohexan umfasst.

3. Verfahren nach Anspruch 2, wobei das nicht mischbare Lösungsmittel Wasser ist.

4. Verfahren nach einem der Ansprüche 1 bis 3, wobei kristallisieren nach dem Solvenz/ Antilsolvenz-Verfahren durchgeführt wird.

5. Verfahren nach Anspruch 4, wobei das im Solvenz/Antilsolvenz-Verfahren verwendete Lösungsmittel aus einer Gruppe ausgewählt ist, die Methanol, n-Propanol, Isopropanol, n-Butanol, sec-Butanol, tert-Butanol und jedwede Kombinationen davon umfasst.

6. Verfahren nach Anspruch 4, wobei das im Solvenz/Antilsolvenz-Verfahren verwendete Antilsolvenz aus einer Gruppe ausgewählt ist, die Aceton, Acetonitril, Petrolether, Cyclohexan, Ethylacetat, Wasser und Heptan umfasst.

7. Verfahren nach Anspruch 1, wobei das Adsorptionsmittel neutrales Aluminiumoxid ist.

8. Verfahren nach Anspruch 1, wobei das eine oder die mehreren Lösungsmittel zum Eluieren des Produkts aus einer Gruppe ausgewählt sind, die Methanol, n-Propanol, iso-Propanol, n-Butanol, sec-Butanol, tert-Butanol, Pentan, Hexan, Heptan, Octan, Ethylacetat und jedwede Kombination von n-Propanol, Isopropanol, n-Butanol, sec-Butanol, tert-Butanol, Pentan, Hexan, Heptan, Octan, Ethylacetat und Wasser umfasst.

9. Verfahren nach Anspruch 1, wobei das geeignete Lösungsmittel n-Butanol ist, wobei zu mit einem nicht mischbaren Lösungsmittel waschen zählt, n-Butanol zuzugeben, so dass eine Produktkonzentration von 5-10 g/kg erhalten wird, und zu behandeln mit Aktivkohle zählt, zu rühren, und filtrieren durch ein Kieselgurbett erfolgt.

10. Verfahren nach Anspruch 1, welches aufweist, die Säule mit einem Gradientengemisch aus Wasser / Methanol für 5-8 Säulenvolumina zu beschicken, nachdem diese ein Säulenvolumen Wasser passiert hat.

11. Verfahren nach Anspruch 1 oder 10, welches aufweist, Pneumocandin B₀ von der Säule mit Methanol zu eluieren.

12. Verfahren nach Anspruch 11, wobei das Methanol 100% Methanol ist.

13. Verfahren nach Anspruch 1, wobei das Pneumocandin B₀ amorphes Pneumocandin B₀ ist.

## Revendications

1. Processus pour purifier la pneumocandine B₀, ayant une ou plusieurs impuretés polaires et une ou plusieurs impuretés non polaires, ledit processus comprenant les actes de :
a) extraction du produit à partir du bouillon de fermentation en extrayant celui-ci avec un solvant approprié et par concentration partielle, dans laquelle le solvant approprié est un alcool sélectionné parmi du n-butanol, sec-butanol, tert-butanol et n-propanol;
b) lavage avec un solvant non miscible ;
c) traitement au charbon de bois ;
d) concentration et filtrage ;
e) cristallisation du filtrat pour obtenir un solide ;
f) chargement du solide dans une colonne avec un adsorbant sélectionné parmi l'alumine ou un gel de silice ;
g) élution d'impuretés en passant un volume de colonne d'eau à travers la colonne ;
h) élution du produit depuis la colonne avec un ou plusieurs solvant(s) pour obtenir de la pneumocandine B0 éluée ; et
i) concentration et cristallisation de la pneumocandine B₀ éluée pour obtenir la pneumocandine B₀.

2. Processus selon la revendication 1, dans lequel le solvant non miscible est sélectionné à partir d'un groupe comprenant de l'eau, de l'éther de pétrole et du cyclohexane.

3. Processus selon la revendication 2, dans lequel le solvant non miscible est de l'eau.

4. Processus selon l'une quelconque des revendications 1 à 3, dans lequel la cristallisation est effectuée par le procédé solvant / anti-solvant.

5. Processus selon la revendication 4, dans lequel le solvant utilisé dans le procédé solvant / anti-solvant est sélectionné à partir d'un groupe comprenant du méthanol, du n-propanol, de l'isopropanol, du n-butanol, du sec-butanol, du tert-butanol et n'importe quelles combinaisons de ceux-ci.

6. Processus selon la revendication 4, dans lequel l'anti-solvant utilisé dans le procédé solvant / anti-solvant est sélectionné à partir d'un groupe comprenant de l'acétone, de l'acétonitrile, de l'éther de pétrole, du cyclohexane, de l'acétate d'éthyle, de l'eau et de l'heptane.

7. Processus selon la revendication 1, dans lequel l'adsorbant est de l'alumine neutre.

8. Processus selon la revendication 1, dans lequel le ou les plusieurs solvants pour éluer le produit est/sont sélectionné(s) à partir d'un groupe comprenant du méthanol, du n-propanol, de l'isopropanol, du n-butanol, du sec-butanol, du tert-butanol, du pentane, de l'hexane, de l'heptane, de l'octane, de l'acétate d'éthyle et n'importe quelle combinaison de n-propanol, isopropanol, n-butanol, sec-butanol, tert-butanol, pentane, hexane, heptane, octane, acétate d'éthyle et eau.

9. Processus selon la revendication 1, dans lequel le solvant approprié est du n-butanol, dans lequel le lavage avec un solvant non miscible comprend l'ajout de n-butanol pour obtenir une concentration de produit de 5 à 10 g/kg, le traitement au charbon de bois comprend de l'agitation et du filtrage est effectué à travers un lit de célite.

10. Processus selon la revendication 1, comprenant l'application d'un mélange de gradient eau / méthanol pour 5 à 8 volumes de colonne après passage d'un volume de colonne d'eau à travers la colonne.

11. Processus selon la revendication 1 ou 10, comprenant l'élution de pneumocandine B₀ depuis la colonne avec du méthanol.

12. Processus selon la revendication 11, dans lequel le méthanol est du méthanol à 100 %.

13. Processus selon la revendication 1, dans lequel la pneumocandine B₀ est de la pneumocandine B₀ amorphe.
